# EUROPÄISCHE PATENTSCHRIFT

(11) **EP 1 979 306 B1**
(45) Veröffentlichungstag und Bekanntmachung des Hinweises auf die Patenterteilung: **17.04.2013**
(21) Anmeldenummer: 07703818.0
(22) Anmeldetag: 12.01.2007
(51) Int. Cl.: C07C 209/78, C07C 209/84, C07C 263/10, C07C 211/50, C07C 265/12

(54) **VERFAHREN ZUR SIMULTANEN HERSTELLUNG VON 4,4'-DIPHENYLMETHANDIAMIN SOWIE DIPHENYLMETHANDIISOCYANAT UND POLYPHENYLENPOLYMETHYLENPOLYISOCYANATEN**
PROCESS FOR SIMULTANEOUSLY PREPARING 4,4'-DIPHENYLMETHANEDIAMINE AND DIPHENYLMETHANE DIISOCYANATE AND POLYPHENYLENEPOLYMETHYLENE POLYISOCYANATES
PROCEDE DE FABRICATION SIMULTANEE DE 4,4'-DIPHENYLMETHANE DIAMINE AINSI QUE DE DIPHENYLMETHANE DIISOCYANATE ET DE POLYPHENYLENE POLYMETHYLENE POLYISOCYANATE

(30) Priorität: 20.01.2006 EP 06100663
(43) Veröffentlichungstag der Anmeldung: 15.10.2008
(73) Patentinhaber: BASF SE, 67056 Ludwigshafen (DE)
(72) Erfinder: BOCK, Michael, 67152 Ruppertsberg (DE); PALLASCH, Hans-Jürgen, 67169 Kallstadt (DE); DEBERDT, Filip, B-2812 Muizen (BE); MOORS, Johnny, B-2040 Antwerpen (BE)
(86) Internationale Anmeldenummer: PCT/EP2007/050277
(87) Internationale Veröffentlichungsnummer: WO 2007/085534

(56) Entgegenhaltungen:
- DE-A1- 1 901 993
- DE-A1- 10 031 540

## Beschreibung

Gegenstand der Erfindung ist ein Verfahren zur simultanen Herstellung von 4,4'-Diphenylmethandiamin sowie Diphenylmethandiisocyanat und Polyphenylenpolymethylenpolyisocyanaten.

Diphenylmethandiisocyanat und Polyphenylenpolymethylenpolyisocyanate, im folgenden auch als MDI bezeichnet, werden in großen Mengen hergestellt und insbesondere zur Herstellung von Polyurethanen eingesetzt. Die großtechnische Herstellung dieser Verbindungen erfolgt insbesondere durch Umsetzung der entsprechenden Amine mit Phosgen. Dabei handelt es sich stets um Gemische von 2- und Mehrkern-Verbindungen. Die Gemische aus 2- und Mehrkern-MDA werden im folgenden auch als Roh-MDA bezeichnet.

Die zur Herstellung von MDI eingesetzten Gemische aus Isomeren des Diphenylmethandiamins und deren höheren Homologen, im folgenden auch als MDA bezeichnet, werden üblicherweise durch sauer katalysierte Umsetzung von Anilin mit Formaldehyd und nachfolgende Neutralisation und Aufarbeitung des Umsetzungsprodukts hergestellt. Dieses aufgearbeitete Produkt wird üblicherweise in einem Lösungsmittel gelöst und in dieser Form zum MDI umgesetzt.

Für bestimmte Einsatzgebiete, beispielsweise als Vernetzer in Kunststoffen oder Lacken, kann auch 2-Kern-MDA eingesetzt werden.

Aus dem Stand der Technik ist eine Reihe von Verfahren zur Abtrennung von 2-Kern-MDA bekannt.

So kann die Abtrennung von 2-Kern-MDA und Reinigung des 4,4'-MDA mittels Extraktion, wie beispielsweise in SU 463 658 beschrieben, mittels Umsetzung mit Metallsalzen, wie in GB 1 169 127 beschrieben, durch Schmelzen, wie in EP 572 030 beschrieben, oder durch Behandlung mit Lösungsmitteln, wie in BE 855 402 und US 4,034,039 beschrieben, erfolgen.

RO 104327 beschreibt die Abtrennung von 2-Kern-MDA mittels Dünnschichtdestillation.

Es ist weiterhin bekannt, das 2-Kern-MDA mittels Destillation von dem Roh-MDA abzutrennen.

DE 1 901 993 beschreibt ein Verfahren zur Herstellung von 4,4'-MDA, bei dem aus einem Gemisch aus 2-Kern-MDA und Mehrkern-MDA das 2-Kern-MDA abdestilliert und daraus durch Kristallisation das 4,4'-MDA abgetrennt wird. Die Destillation erfolgt bei 2 Torr und 220 bis 230°C.

In DE 100 31 540 wird ein Verfahren zur Abtrennung von 2,2'-MDA und 2,4'-MDA vom Roh-MDA beschrieben. Dazu kann eine Destillationskolonne mit mindestens 40 Trennstufen eingesetzt werden. Die Destillation erfolgt bei einem Temperaturverlauf von 180-280°C, einem Kopfdruck von 0,1-10 mbar und einem Sumpfdruck von 8-20 mbar. Zur Reduzierung der Druckverluste werden druckverlustarme Gewebepackungen eingesetzt. Das von 2,2'- und 2,4'-MDA befreite Roh-MDA wird mit Phosgen zu MDI umgesetzt, das abgetrennte 2,2'- und 2,4'-MDA wird in die Kondensationsstufe zurückgeführt.

Nachteilig bei allen bisherigen Verfahren zur Abtrennung von 2-Kern-MDA und der Trennung der Isomeren ist es, dass dazu ein apparativ aufwendiges separates Verfahren nötig war, bei dem die anfallenden Nebenprodukte zumeist entsorgt werden mussten.

Aufgabe der vorliegenden Erfindung war es, ein Verfahren zur Herstellung von 2-Kern-MDA mit einem hohen Anteil, insbesondere von mindestens 80 %, an 4,4'-MDA bereitzustellen, das einen geringen verfahrenstechnischen Aufwand erfordert. Außerdem sollte es möglich sein, auf einfachem Wege 2-Kern-MDA mit unterschiedlichen Mischungen zu erzeugen.

Die Aufgabe konnte gelöst werden durch ein simultanes Verfahren zur Herstellung von MDI und 2-Kern-MDA.

Gegenstand der Erfindung ist demzufolge ein Verfahren zur simultanen Herstellung von 4,4'-Diphenylmethandiamin sowie Diphenylmethandiisocyanat und Polyphenylenpolymethylenpolyisocyanaten, umfassend die Schritte
a) Herstellung einer Mischung aus Diphenylmethandiamin und Polyphenylenpolymethylenpolyaminen durch saure Kondensation von Anilin und Formaldehyd und nachfolgende Aufarbeitung der Mischung,
b) Abtrennung eines Teils der in Schritt a) hergestellten Mischung,
c) Destillation der in Schritt b) abgetrennten Mischung in einer Kolonne,
d) Rückführung des Sumpfprodukts aus Schritt c) zum Endprodukt aus Schritt a) und Kondensation des Kopfprodukts aus Schritt c)
e) Destillation des Kopfprodukts aus Schritt c) in einer Kolonne,
f) Rückführung des Kopfprodukts aus Schritt e) zum Endprodukt aus Schritt a),
g) Gewinnung des als Sumpfprodukt in Schritt e) anfallenden 4,4'-Diphenylmethandiamins,
h) Umsetzung der Mischung aus Schritt a) mit Phosgen und nachfolgende Aufarbeitung des Umsetzungsprodukts.

Das Verfahren kann unterschiedlich ausgestaltet werden. In einer Ausführungsform des Verfahrens können alle Schritte den Verfahren kontinuierlich betrieben werden.

Falls nicht ständig 2-Kern-MDA erzeugt werden soll, ist es möglich, die Verfahrensschritte b) bis g) zeitweise stillzulegen und in dieser Zeit alles in Verfahrenschritt a) hergestellte Produkt mit Phosgen umzusetzen.

Die Destillation in den Verfahrensschritten c) und e) kann vorzugsweise in zwei unterschiedlichen Kolonnen vorgenommen werden. Zur Verdampfung der Brüden können produktschonende Verdampfer mit kurzer Verweilzeit, insbesondere Fallfilmverdampfer eingesetzt werden. Es ist prinzipiell auch möglich, beide Schritte in einer Trennwandkolonne durchzuführen.

Eine besonders schonende Destillation des 4,4'-MDA kann bei Sumpfdrücken < 8 mbar, insbesondere < 6 mbar erreicht werden. Dies gelingt bevorzugt durch Verwendung von besonders druckverlustarmen, strukturierten Packungselementen und Flüssigkeitsverteilern. Der Druckverlust über alle Packungselemente, Sammler, Verteiler und den Kondensator beträgt in der Trennkolonne von Schritt c) vorzugsweise 0,5-3 mbar, besonders bevorzugt weniger als 0,8 mbar. In der Trennkolonne von Schritt e) beträgt der Druckverlust über alle Packungselemente, Sammler, Verteiler und den Kondensator vorzugsweise weniger als 5 mbar, besonders bevorzugt weniger als 3,5 mbar. Der Druck am Kopf der Kolonnen in den Schritten c) und e) beträgt vorzugsweise 3 mbar abs.

Die benötigte Trennstufenzahl beträgt in der Trennkolonne von Schritt c) vorzugsweise 1 bis 5 besonders bevorzugt 2-3 und in der Trennkolonne von Schritt e) vorzugsweise 9-20, besonders bevorzugt 10-13 theoretische Trennstufen.

Die Sumpftemperaturen betragen in beiden Kolonnen vorzugsweise 200-250°C, bevorzugt 220-240°C. In der Kolonne von Schritt c) beträgt die Sumpftemperatur besonders bevorzugt 235 bis 240°C. Die Kopftemperatur beträgt bei der Kolonne von Schritt c) vorzugsweise 200 bis 210 °C und bei der Kolonne von Schritt e) vorzugsweise 190 bis 210°C.

Bei den genannten Bedingungen kann am Ende von Schritt c) 4,4'-MDA mit einer Reinheit von mindestens 79 Gew.-% und am Ende von Schritt e) 4,4-,MDA mit einer Reinheit von mindestens 98 % gewonnen werden. Falls ein 2-Kern-MDA mit einer anderen Isomerenverteilung bereitgesellt werden soll, kann dies bevorzugt durch Mischung der Produktströme erreicht werden. Bevorzugt wird dabei das Sumpfprodukt aus Schritt e) mit dem Kopfprodukt aus Schritt e) oder besonders bevorzugt mit dem Kopfprodukt aus Schritt c) vermischt.

Die Reinheit des in Schritt e) anfallenden 4,4'-MDA ist völlig unabhängig vom Gehalt an 2-Kern-MDA im Reaktionsprodukt aus Schritt a). Selbst bei geringen Anteilen, beispielsweise von 50 bis 60 Gew.-% oder bei stark schwankenden Anteilen an 2-Kern-MDA im Produkt aus Schritt a) kommt es zu keiner Beeinträchtigung der übrigen Verfahrensschritte.

In Schritt a) des erfindungsgemäßen Verfahrens wird wie üblich Anilin mit Formaldehyd zu MDA unter Verwendung saurer Katalysatoren umgesetzt. Dieses Verfahren ist allgemein bekannt und beispielsweise in DE 100 31 540 beschrieben durch die Variation des Verhältnisses von Säure zu Anilin und von Formaldehyd zu Anilin kann der Anteil des 2-Kern-Produkts im Roh-MDA je nach Wunsch eingestellt werden.

Die Menge des in Schritt b) abgenommenen Teilstroms ist abhängig von der benötigten Menge des 4,4'-MDA. Um eine zu starke Veränderung der Zusammensetzung des in Schritt h) entstehenden MDI zu vermeiden, sollte die abgezogene Menge nicht höher als 20 Gew.-%, bevorzugt < 15 Gew.-% .sein. Man kann einer Beeinflussung der Zusammensetzung des Produkts aus Schritt h) auch dadurch entgegenwirken, dass man in Schritt a) die Reaktionsbedingungen so auswählt, dass ein hoher Anteil an 4,4'-MDA gebildet wird. Dies lässt sich durch eine Steigerung des Verhältnisses von Säure zu Anilin erreichen. Ein höheres Verhältnis bedeutet, dass mehr 4,4'-MDA als 2,4'-MDA gebildet wird. Der 2-Kern MDA Gehalt steigt dann insgesamt leicht. Durch eine Erhöhung des Verhältnisses von Anilin zu Formaldehyd kann insgesamt mehr 2-Kern-MDA gebildet werden. Vorzugsweise beträgt der Anteil des 2-Kerm-MDA im Produkt aus Schritt a) zwischen 50 und 60 Gew.-%.

Im folgenden soll eine bevorzugte Ausgestaltung des erfindungsgemäßen Verfahrens beschrieben werden.

Aus einem MDA-Gemisch aus der sauer katalysierten Kondensation von Anilin und Formaldehyd wird ein Teilstrom von größer 0 bis 20 Gew.-% abgetrennt, je nachdem, in welchem Lastbereich die Anlage läuft. Dieser hat zumeist eine Temperatur in Bereich zwischen 120 und 200°C. In einem Wärmetauscher wird er auf 150-220°C erwärmt.

Der Feedstrom wird zur Abtrennung des 2-Kern-MDI einer Kolonne mit einer strukturierten Packung zugeführt, wobei er zuvor mit dem Sumpfprodukt dieser Kolonne vermischt wird. Das so erhaltene Gemisch wird im Sumpf der Kolonne mit einem Fallfilmverdampfer auf 235-240 °C erhitzt.

Als Packung können handelsübliche strukturierte Packungen, beispielsweise der Firmen Sulzer oder Montz benutzt werden.

Die leichter flüchtigen Komponenten, im wesentlichen 2-Kern-MDA, gehen über den Kopf der Kolonne, die schwerer flüchtigen Komponenten, im wesentlichen Mehrkern-MDA mit einem Anteil 2-Kern-MDA, verbleiben im Sumpf der Kolonne und werden als PMDA-Gemisch ausgetragen. Ein Teil des Sumpfproduktes wird ausgetragen und dem PMDA-Strom zur Phosgenierung zugeführt. Der Rest des Sumpfprodukts wird, wie beschrieben, mit dem Feedstrom vermischt und erneut der Kolonne zugeführt. Im Kopf der Kolonne herrscht ein Druck von ca. 3 mbar abs.

Das ausgetragene Sumpfprodukt gibt die Wärme teilweise über einen Wärmetauscher an den Feedstrom ab.

Das Kopfprodukt wird in einem integrierten Wärmetauscher praktisch vollständig kondensiert. In einem nachgeschalteten Wärmetauscher werden aus der Gasphase restliche Spuren an MMDA und Anilin auskondensiert. Die Kondensation findet bei 90 bis 100°C statt. Es fällt ein Abgasstrom an, der zu ca. 97 % aus Leckluft sowie ca. 3 % organischen Komponenten besteht.

Das im ersten Wärmetauscher kondensierte Kopfprodukt wird in zwei Teilströme getrennt.

Der erste Strom bildet den Feed einer weiteren Kolonne, in der das 2-Kern-MDA destilliert wird. (Schritt e). Im Kopf dieser Kolonne herrscht ein Druck von ca. 3 mbar abs. Die Kolonne ist ebenfalls mit einer strukturierten Packung gefüllt. Zum Aufheizen des Feeds der Kolonne wird ein Fallfilmverdampfer verwendet, der mit Dampf beaufschlagt ist. Die Kolonne hat eine Sumpftemperatur von ca. 220°C. Das leichter siedende Gemisch von 2,2'-, 2,4'- und teilweise noch 4,4'-MDA wird als Kopfprodukt in einem nachgeschalteten Wärmetauscher der K2 praktisch vollständig kondensiert.

Das kondensierte Kopfprodukt wird ebenfalls dem PMDA-Strom zur Phosgenierung zugeführt.

Das Sumpfprodukt der Kolonne von Schritt e) enthält ca. 98 % 4,4'-MDA und kann direkt verkauft werden.

Zur Einstellung anderer Isomerenverhältnisse als 98 % 4,4'-MDA zum restlichen 2-Kern MDA kann das Sumpfprodukt aus der zweiten Kolonne mit einem Teilstrom des Kopfprodukts aus der ersten Kolonne vermischt werden.

### Beispiel 1

Herstellung von reinem 4,4'-Diphenylmethandiamin in hohem Gehalt an 4,4'-Isomeren (98,0 %)

Aus dem PMDA Strom zur Phosgenierung von 20t/h (a) werden kontinuierlich 1750 kg/h (8,75 Gew.-%) zur Diphenylmethandiamin-Destillation (b) abgezweigt. Darin befinden sich 10 Gew.-% 2,4'-Diphenylmethandiamin, 47,3 Gew.-% 4,4'-Diphenylmethandiamin und 0,6 Gew.-% 2,2'-Diphenylmethandiamin sowie restlichen Bestandteilen von 3-Kern und Mehrkernverbindungen von Polyphenylmethandiamin.

Die Mischung wird auf 150°C vorgewärmt und dann in den Umpumpkreislauf eines Fallfilmverdampfers der Kolonne c) gepumpt. Die Eintrittstemperatur in die Kolonne c) beträgt 220°C.

Am Kopf der Kolonne c) wird eine Mischung von 785 kg/h 2-Kern Isomeren mit 80 Gew.-% 4,4'-Diphenylmethandiamin abgezogen. Ein geringer Teil an nicht kondensierbaren Komponenten geht mit ins Abgas.

Am Kolonnensumpf fallen dementsprechend 960 kg/h 3- und Mehrkernkomponenten, sowie geringe Teile an 2-Kern Diphenylmethandiamin (ca. 24 Gew.-%) an, die zur Phosgenierung gehen (d).

Kolonne c) wird bei 4mbar Kopfdruck und 240°C Sumpftemperatur betrieben.

Das Kopfkondensat aus c) wird in der Kolonne e) rektifiziert. Diese Kolonne wird bei 4 mbar Kopfdruck und 230°C Sumpftemperatur betrieben. Am Sumpf fallen 455 kg/h einer Mischung beinhaltend 98,0 Gew.-% 4,4'Diphenylmethandiamin an (g). Am Kopf der Kolonne wird der unscharfe Schnitt aus 2-Kern Diphenylmethandiamin mit 53 Gew.-% 4,4'-Diphenylmethandiamin, gesamt 330 kg/h nach a) zurückgeführt

### Beispiel 2

Simultane Herstellung von zwei 4,4'-Diphenylmethandiamin-Qualitäten: 4,4'-Diphenylmethandiamin 98,0 Gew.-% und 4,4'-Diphenylmethandiamin 90 Gew.-%

Aus dem PMDA Strom zur Phosgenierung (a) von 20t/h werden kontinuierlich 1750 kg/h (8,75 Gew.-%) zur Diphenylmethandiamin Destillation abgezweigt. Darin befinden sich 10 Gew.-% 2,4'-Diphenylmethandiamin, 47,3 Gew.-% 4,4'-Diphenylmethandiamin und 0,6 Gew.-% 2,2'-Diphenylmethandiamin sowie restlichen Bestandteilen von 3 oder Mehrkernverbindungen.

Die Mischung wird auf 150°C vorgewärmt und dann in den Umpumpkreislauf des Fallfilmverdampfers der Kolonne c) gepumpt. Die Eintrittstemperatur in die Kolonne c) beträgt 220°C.

Am Kopf der Kolonne c) wird eine Mischung von 785 kg/h 2-Kern Isomeren mit 80 Gew.-% 4,4'-Diphenylmethandiamin abgezogen. Ein geringer Teil an nicht kondensierbaren Komponenten geht mit ins Abgas. Am Kolonnensumpf fallen dementsprechend 960 kg/h 3- und Mehrkernkomponenten, sowie geringe Teile an 2-Kern Diphenylmethandiamin (ca. 24 Gew.-%) an, die zur Phosgenierung gehen (d). Kolonne c) wird bei 4 mbar Kopfdruck und 240°C Sumpftemperatur betrieben.

Das Kopfkondensat aus c) wird nun nur zum Teil in der Kolonne e) rektifiziert. Von den 785 kg/h werden 695 kg/h in die Kolonne gefahren. Die Kolonne (e) wird bei 4 mbar Kopfdruck und 230°C Sumpftemperatur betrieben. Am Sumpf der Kolonne fallen 399 kg/h einer Mischung mit 98,0% 4,4'Diphenylmethandiamin an (Produkt 1).

30 Gew.-% des Stroms aus dem Kolonnensumpf werden jetzt mit dem nicht in die Kolonne geführten Produkt (90 kg/h) vermischt. Damit ergibt sich eine weitere Mischung mit 90 % 4,4'-Diphenylmethandiamin (Produkt 2).

Als Ergebnis erhält man zwei Produktqualitäten aus einem Strom a), nämlich 279 kg/h 98 Gew.-% 4,4'-Diphenylmethandiamin und 209 kg/h mit 90,3 Gew.-% 4,4'-Diphenylmethandiamin.

## Patentansprüche

1. Verfahren zur simultanen Herstellung von 4,4'-Diphenylmethandiamin sowie Diphenylmethandiisocyanat und Polyphenylenpolymethylenpolyisocyanaten, umfassend die Schritte
a) Herstellung einer Mischung aus Diphenylmethandiamin und Polyphenylenpolymethylenpolyaminen durch saure Kondensation von Anilin und Formaldehyd und nachfolgende Aufarbeitung der Mischung,
b) Abtrennung eines Teils der in Schritt a) hergestellten Mischung,
c) Destillation der in Schritt b) abgetrennten Mischung in einer Kolonne,
d) Rückführung des Sumpfprodukts aus Schritt c) zum Endprodukt aus Schritt a) und Kondensation des Kopfprodukts aus Schritt c)
e) Destillation des Kopfprodukts aus Schritt c) in einer Kolonne,
f) Rückführung des Kopfprodukts aus Schritt e) zum Endprodukt aus Schritt a),
g) Gewinnung des als Sumpfprodukt in Schritt e) anfallenden 4,4'-Diphenylmethandiamins,
h) Umsetzung der Mischung aus Schritt a) mit Phosgen und nachfolgende Aufarbeitung des Umsetzungsprodukts.

2. Verfahren nach Anspruch 1, **dadurch gekennzeichnet, dass** alle Schritte des Verfahrens kontinuierlich durchgeführt werden.

3. Verfahren nach Anspruch 1, **dadurch gekennzeichnet, dass** das Gemisch aus Diphenylmethandiamin und Polyphenylen-polymethylen-polyaminen bei einer Temperatur im Bereich zwischen 160 und 180 °C in die Kolonne in Schritt c) eintritt.

4. Verfahren nach Anspruch 1, **dadurch gekennzeichnet, dass** am Kopf der Kolonne in Schritt c) ein Druck von 1-5 mbar abs herrscht.

5. Verfahren nach Anspruch 1, **dadurch gekennzeichnet, dass** am Kopf der Kolonne in Schritt c) eine Temperatur von 200 bis 210°C herrscht.

6. Verfahren nach Anspruch 1, **dadurch gekennzeichnet, dass** der Druckverlust der Kolonne in Schritt c) über alle Packungselemente, Sammler, Verteiler und den Kondensator weniger als 0,9 mbar beträgt.

7. Verfahren nach Anspruch 1, **dadurch gekennzeichnet, dass** die Kolonne in Schritt c) eine Sumpftemperatur von 235 bis 240°C hat.

8. Verfahren nach Anspruch 1, **dadurch gekennzeichnet, dass** die Kolonne in Schritt c) eine Trennleistung von 2-3 theoretischen Trennstufen besitzt.

9. Verfahren nach Anspruch 1, **dadurch gekennzeichnet, dass** die Kolonnen in Schritt c) und e) eine druckverlustarme strukturierte Packung aufweisen.

10. Verfahren nach Anspruch 1, **dadurch gekennzeichnet, dass** am Kopf der Kolonne in Schritt e) ein Druck von 1-4 mbar abs herrscht.

11. Verfahren nach Anspruch 1, **dadurch gekennzeichnet, dass** am Kopf der Kolonne in Schritt e) eine Temperatur von 190-210°C herrscht.

12. Verfahren nach Anspruch 1, **dadurch gekennzeichnet, dass** der Druckverlust der Kolonne in Schritt e) über alle Packungselemente, Sammler, Verteiler und den Kondensator weniger als 3,5 mbar beträgt.

13. Verfahren nach Anspruch 1, **dadurch gekennzeichnet, dass** die Kolonne in Schritt e) eine Sumpftemperatur von 220-240°C hat.

14. Verfahren nach Anspruch 1, **dadurch gekennzeichnet, dass** die Kolonne in Schritt e) eine Trennleistung von 10-13 theoretischen Trennstufen besitzt.

15. Verfahren nach Anspruch 1, **dadurch gekennzeichnet, dass** ein Teil Kopfprodukt der Kolonne aus Schritt e) mit einer beliebigen Menge des Sumpfproduktes der Kolonne aus Schritt c) vermischt wird.

## Claims

1. A process for the simultaneous preparation of 4,4'-diphenylmethanediamine and also diphenylmethane diisocyanate and polyphenylenepolymethylene polyisocyanates, which comprises the steps
a) preparation of a mixture of diphenylmethanediamine and polyphenylenepolymethylenepolyamines by acid condensation of aniline and formaldehyde and subsequent work-up of the mixture,
b) splitting-off of part of the mixture prepared in step a),
c) distillation of the mixture separated off in step b) in a column,
d) recirculation of the bottom product from step c) to the end product from step a) and condensation of the overhead product from step c),
e) distillation of the overhead product from step c) in a column,
f) recirculation of the overhead product from step e) to the end product from step a),
g) isolation of the 4,4'-diphenylmethanediamine obtained as bottom product in step e),
h) reaction of the mixture from step a) with phosgene and subsequent work-up of the reaction product.

2. The process according to claim 1, wherein all steps of the process are carried out continuously.

3. The process according to claim 1, wherein the mixture of diphenylmethanediamine and polyphenylenepolymethylenepolyamines enters the column in step c) at a temperature in the range from 160 to 180°C.

4. The process according to claim 1, wherein a pressure of 1-5 mbar abs. prevails at the top of the column in step c).

5. The process according to claim 1, wherein a temperature of from 200 to 210°C prevails at the top of the column in step c).

6. The process according to claim 1, wherein the pressure drop over all packing elements, collectors, distributors and the condenser of the column in step c) is less than 0.9 mbar.

7. The process according to claim 1, wherein the column in step c) has a temperature at the bottom of from 235 to 240°C.

8. The process according to claim 1, wherein the column in step c) has a separation power of 2-3 theoretical plates.

9. The process according to claim 1, wherein the columns in steps c) and e) have a low-pressure-drop structured packing.

10. The process according to claim 1, wherein a pressure of 1-4 mbar abs. prevails at the top of the column in step e).

11. The process according to claim 1, wherein a temperature of 190-210°C prevails at the top of the column in step e).

12. The process according to claim 1, wherein the pressure drop over all packing elements, collectors, distributors and the condenser of the column in step e) is less than 3.5 mbar.

13. The process according to claim 1, wherein the column in step e) has a temperature at the bottom of 220-240°C.

14. The process according to claim 1, wherein the column in step e) has a separation power of 10-13 theoretical plates.

15. The process according to claim 1, wherein part of the overhead product from the column in step e) is mixed with a desired amount of the bottom product from the column in step c).

## Revendications

1. Procédé de fabrication simultanée de 4,4'-diphénylméthane-diamine, ainsi que de diisocyanate de diphénylméthane et de polyisocyanates de polyphénylène-polyméthylène, comprenant les étapes :
a) la fabrication d'un mélange de diphénylméthane-diamine et de polyphénylène-polyméthylène-polyamines par condensation acide d'aniline et de formaldéhyde et traitement ultérieur du mélange,
b) la séparation d'une partie du mélange fabriqué à l'étape a),
c) la distillation du mélange séparé à l'étape b) dans une colonne,
d) le recyclage du produit de fond de l'étape c) dans le produit final de l'étape a) et la condensation du produit de tête de l'étape c),
e) la distillation du produit de tête de l'étape c) dans une colonne,
f) le recyclage du produit de tête de l'étape e) dans le produit final de l'étape a),
g) l'obtention de la 4,4'-diphénylméthane-diamine formée en tant que produit de fond à l'étape e),
h) la mise en réaction du mélange de l'étape a) avec du phosgène et le traitement ultérieur du produit de réaction.

2. Procédé selon la revendication 1, **caractérisé en ce que** toutes les étapes du procédé sont réalisées en continu.

3. Procédé selon la revendication 1, **caractérisé en ce que** le mélange de diphénylméthane-diamine et de polyphénylène-polyméthylène-polyamines entre dans la colonne à l'étape c) à une température dans la plage comprise entre 160 et 180 °C.

4. Procédé selon la revendication 1, **caractérisé en ce qu'**une pression de 1 à 5 mbar abs règne à la tête de la colonne à l'étape c).

5. Procédé selon la revendication 1, **caractérisé en ce qu'**une température de 200 à 210 °C règne à la tête de la colonne à l'étape c).

6. Procédé selon la revendication 1, **caractérisé en ce que** la perte de charge de la colonne à l'étape c) sur tous les éléments de garnissage, le collecteur, le distributeur et le condensateur est inférieure à 0,9 mbar.

7. Procédé selon la revendication 1, **caractérisé en ce que** la colonne à l'étape c) a une température de fond de 235 à 240 °C.

8. Procédé selon la revendication 1, **caractérisé en ce que** la colonne à l'étape c) présente une puissance de séparation de 2 à 3 étapes de séparation théoriques.

9. Procédé selon la revendication 1, **caractérisé en ce que** les colonnes à l'étape c) et e) comprennent un garnissage structuré à faible perte de charge.

10. Procédé selon la revendication 1, **caractérisé en ce qu'**une pression de 1 à 4 mbar abs règne à la tête de la colonne à l'étape e).

11. Procédé selon la revendication 1, **caractérisé en ce qu'**une température de 190 à 210 °C règne à la tête de la colonne à l'étape e).

12. Procédé selon la revendication 1, **caractérisé en ce que** la perte de charge de la colonne à l'étape e) sur tous les éléments de garnissage, le collecteur, le distributeur et le condensateur est inférieure à 3,5 mbar.

13. Procédé selon la revendication 1, **caractérisé en ce que** la colonne à l'étape e) a une température de fond de 220 à 240 °C.

14. Procédé selon la revendication 1, **caractérisé en ce que** la colonne à l'étape e) présente une puissance de séparation de 10 à 13 étapes de séparation théoriques.

15. Procédé selon la revendication 1, **caractérisé en ce qu'**une partie du produit de tête de la colonne de l'étape e) est mélangée avec une quantité quelconque du produit de fond de la colonne de l'étape c).
